Europäisches Patentamt

European Patent Office

Office européen des brevets

(19)

(11) Publication number : **0 204 676**
**B1**

(12)  **EUROPEAN PATENT SPECIFICATION**

(45) Date of publication of patent specification :
03.01.90

(51) Int. Cl.⁵ : **A 61 B   6/14**

(21) Application number : **86850184.2**

(22) Date of filing : **22.05.86**

(54) **Panoramic tomography X-ray apparatus particularly for dental photography.**

(30) Priority : **31.05.85 FI 852208**

(43) Date of publication of application :
**10.12.86 Bulletin 86/50**

(45) Publication of the grant of the patent :
**03.01.90 Bulletin 90/01**

(84) Designated contracting states :
**AT BE CH DE FR GB IT LI LU NL SE**

(56) References cited :
**DE-A- 2 846 876**
**DE-A- 3 125 243**
**DE-A- 3 434 369**
**FR-A- 2 369 824**
**FR-A- 2 373 268**
**US-A- 3 737 660**

(73) Proprietor : **PLANMECA OY**
**Mekaanikonkatu 5**
**SF-00810 Helsinki (FI)**

(72) Inventor : **Virta, Arto**
**Pohjolantie 15**
**FI-01260 Vantaa (FI)**
Inventor : **Strömmer, Pekka**
**Ilmakuja 5 F 49**
**FI-02210 Espoo (FI)**

(74) Representative : **Onn, Thorsten et al**
**AB STOCKHOLMS PATENTBYRA Box 3129**
**S-103 62 Stockholm (SE)**

Jouve, 18, rue St-Denis, 75001 Paris, France

**Description**

The present invention relates to a panoramic tomography X-ray apparatus particularly for dental photography which apparatus comprises a fixed frame, to which another frame (rotary frame) having an X-ray tube at one end and a film cartridge device for X-ray film at the other end, is suspended with bearings, and between which X-ray tube and film cartridge the patient's object to be photographed can be positioned, and which apparatus has a drive mechanism by means of which said suspended rotary frame is rotated in a plane, preferably in the horizontal plane, for taking a panoramic X-ray photograph.

The design and operation principle of most panoramic X-ray apparatuses is such that the X-ray is turned around the patient's head in such a way that the dental arch will be photographed as a flat picture on a moving film.

In order to make the object being photographed sharp and the structures in front of the object and behind the object invisible by « fogging » them out of focus, the lateral velocity of the film with regard to the ray bundle must be equal to the sweep velocity of the ray bundle in the object multiplied with the magnification ratio. The magnification is determined by the ratio of the distance between the focus and the film to the distance between the focus and the object.

The thickness of the layer being photographed sharply is directly proportional to the distance of the instantaneous center of rotation from the film level, and inversely proportional to the magnification and to the width of the ray bundle. From the point of view of how the object will be represented the only important thing is how the focus, the object and the film level are located with regard to each other. The instantaneous center of rotation has significance only through the sweep velocity.

On account of what has been said above it is possible to form the basic equation of panoramic photography :

$v_1/v_0 = L_1/L_0,$

$v_0 = Wr,$ where

$L_0$ = the distance from the focus F to the point being photographed at a given moment

$L_1$ = the distance from the focus F to the film level

W = the angular velocity of the rotary movement around the center of rotation

r = the distance of the point being photographed from the instantaneous center of rotation

$v_1$ = the velocity of an image point on the picture level (film level)

The most important objects of photography by means of a panoramic tomography X-ray apparatus are the dental arch and the temporomandibular joints (there are other important subjects, too). In order that the apparatus would produce as useful projection as possible of the object, the mechanically least problematic arrangement, the fixed center of rotation, must be abandoned. The way to move the position of the center of rotation depends, among other things, on the following factors :

orthogonality, which is aimed at the prevention of adjacent teeth at any point of the dental arch from being photographed on top of each other ; therefore the ray bundle must sweep the object as perpendicularly as possible, i. e. orthogonally against the dental arch ;

constant magnification, i. e. the magnification must be the same all over the dental arch, for which the distance from the layer being photographed to the film level is maintained constant throughout the whole rotation ;

evenness of the movement, which has been difficult to maintain, as it has been necessary to move the instantaneous center of rotation during the exposure. The projection must be such that the instantaneous center of rotation may move without incontinuities which could create excessive accelerations thus harming the quality of the picture ;

minimization of the radiation load to the patient, for which the projection must be such that excessive radiation will not be applied to a single spot of the patient.

Except that the X-ray source of the apparatus and the rotary mechanism of the film cartridge create a projection which meets the requirements specified above, it should be possible to manufacture them with reasonable expenses so that there will be no play or other inaccuracies detrimental to the photography. In order to create the desired projection, the mechanism must therefore move the center of rotation in the horizontal plane. The mechanism must also vertically support the whole equipment ; therefore, in order to provide a good photograph, also the vertical bearing system must be stable and without play.

In the Prior Art, said orthogonality has been satisfactorily maintained in the dental arch area. Almost no attention has been paid to the temporomandibular joints. With the commercially available apparatuses it is not possible to keep the magnification ratio constant, which shortcoming can be seen in the tomography exposures in such a way that the front part of the dental arch and/or the whole dental arch are photographed with a noticeably higher magnification ratio that the temporomandibular joints. The reason for this is that in the known apparatuses the film cartridge comes in the side area much closer to the object than in the front area.

In the Prior Art, the rotary movement is also such that, during the whole exposure, the center of rotation is located in the area outlined by the temporomandibular joints or near this area, which results in a high local radiation load in this area. For the same reason the temporomandibular joints are photographed in an oblique angle, which is, for diagnostic reasons, an obvious drawback.

The technological level linked with the invention will now be described in detail with reference to patent literature having most in common with the invention.

The Finnish patent application No. 833754 (Instrumentarium Oy) depicts a panoramic tomography equipment, in which the constant magnification problem is solved in one manner. This reference however takes no attitude to how the projection is produced. A solution in accordance with the GB patent No. 1 594 499, referred to in the above application, produces a projection, in which the temporomandibular joints are photographed in a highly oblique angle (not orthogonally). In an apparatus in accordance with the above application the movement parallel with the ray bundle, required by constant magnification, is created by a linear bearing system, which is constructively a difficult and expensive solution.

The Finnish patent application No. 783233 (equivalent US patent 4 264 820) deals with an X-ray method and apparatus, in which attention has been paid to the constant magnification problem. Even this reference has no views on the projection to the temporomandibular joint area; the orthogonality is examined only in the dental arch area. Two systems are described for attaining constant magnification; in these systems the following drawbacks can be perceived:

so-called revolving slides move in grooves, which makes it very difficult, even impossible, to make them work without play;

the stability of the apparatus in the horizontal plane is questionable in such points of the rotary movement in which the revolving slide guide grooves are in an unfavourable angle towards each other;

a method of transmitting rotary movement leads to impure rotation of the drive wheel (axial displacement);

in the special arrangement described in the reference, in which one of the revolving slide grooves is replaced with a crank, the control of the revolving cranks in the grooves is critical from the point of view of the result of the exposure, as the revolving slide does not stably follow either one of the groove edges;

in the last-mentioned special arrangement of the above reference the transmission of the rotary movement can be arranged so that the drive will be pure as the drive wheel runs along a circular track, but the design will be complex, bulky and expensive.

A general object of the present invention is to provide such an apparatus in which the problems discussed above are solved by means of an arrangement that is mechanically simple and feasible.

A specific object of the invention is to provide such an apparatus in which there are no detrimental mechanical plays or similar inaccuracies.

An object of the invention is to provide such an apparatus in which the orthogonality described above is carried out both in the dental arch area and in the rear part of the jawbone up to the temporomandibular joints with good sharpness.

An object of the invention is to provide such an apparatus in which the magnification is constant over the whole image field.

An additional object of the invention is to provide such an apparatus in which the film can be brought closer to the patient in order to reduce the magnification ratio without any risk of the film cartridge hitting the patient.

An additional object of the invention is to provide such a device in which the maximum local radiation load can be made essentially lower than before.

The objects of the invention are met by the features of the device according to claim 1.

The invention will now be described in detail, with reference to some exemplary embodiments illustrated in the figures in the accompanying drawings, with no intention to restrict the invention to the details of these embodiments.

Figure 1 shows the projection produced by an apparatus in accordance with the invention.

Figure 2 is a schematic plan view of the drive mechanism in an end position.

Figure 3 shows the same drive mechanism in an intermediate position.

Figure 4 shows the drive mechanism shown in figures 2 and 3 in the middle position.

Figure 5 is a central vertical section of a mechanism and apparatus in accordance with the invention; figure 5 is also the section V-V in figure 4 showing the mechanism in the middle position.

Figure 6 is section VI-VI in figure 5.

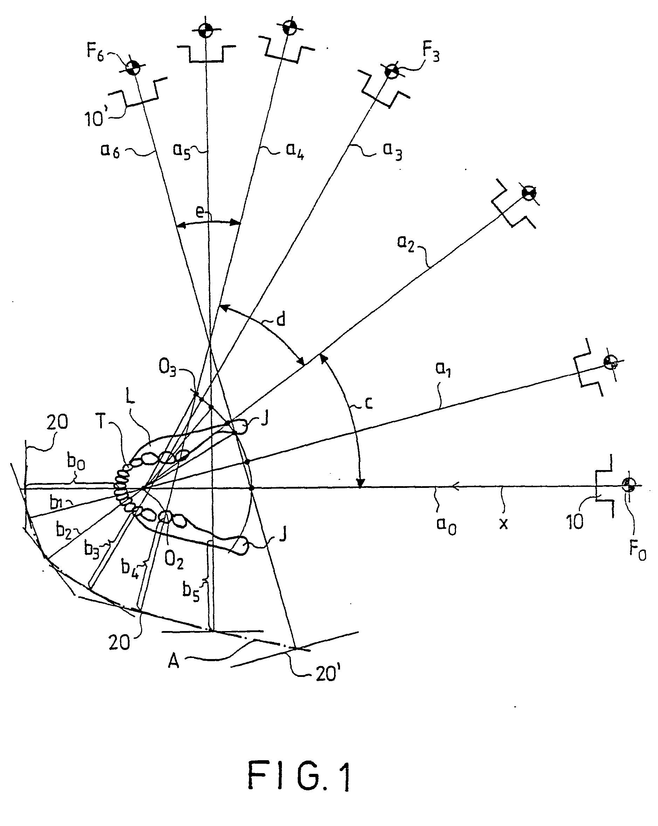

Figure 1 illustrates the photographing geometry and projection accomplished with the apparatus in accordance with the invention. The X-ray tube is marked with reference number 10, its focus in different positions with references $F_0$ to $F_6$. An X-ray tube 10 transmits an X-ray bundle X through the teeth T and the jawbone L onto a film 20 along the line a. In figure 1, the passage of the X-ray X is shown in seven different positions $a_0$ to $a_6$. In an end position, the X-ray tube is marked with 10′, the film with 20′ and the focus of the tube with $F_6$. The joints of the jawbone L are marked with J.

In the front area of the dental arch, which is represented by the space between the rays $a_0$ and $a_2$ (sector c), a rotary frame (an arm) 11, carrying the X-ray tube 10 on its one end and the film 20 at its other end, rotates in the horizontal plane around the vertical axis $O_2$. Between the rays $a_2$ and $a_4$ (sector d), the center of rotation smoothly moves along a curved path to the vertical axis $O_3$, after which the vertical axis further draws away from the center axis $a_0$ (C-C). With the geometry illustrated in figure 1, the orthogonality of the representation comes true with good accuracy besides in the front dental arch area and in the side areas also in the rear part of the jawbone L up to the joints J. Magnification will also be constant in the whole image field; this is proved by for instance by the fact that the distance $b_0$-$b_5$ of the film from the photographed layer is precisely enough constant over the whole path. As

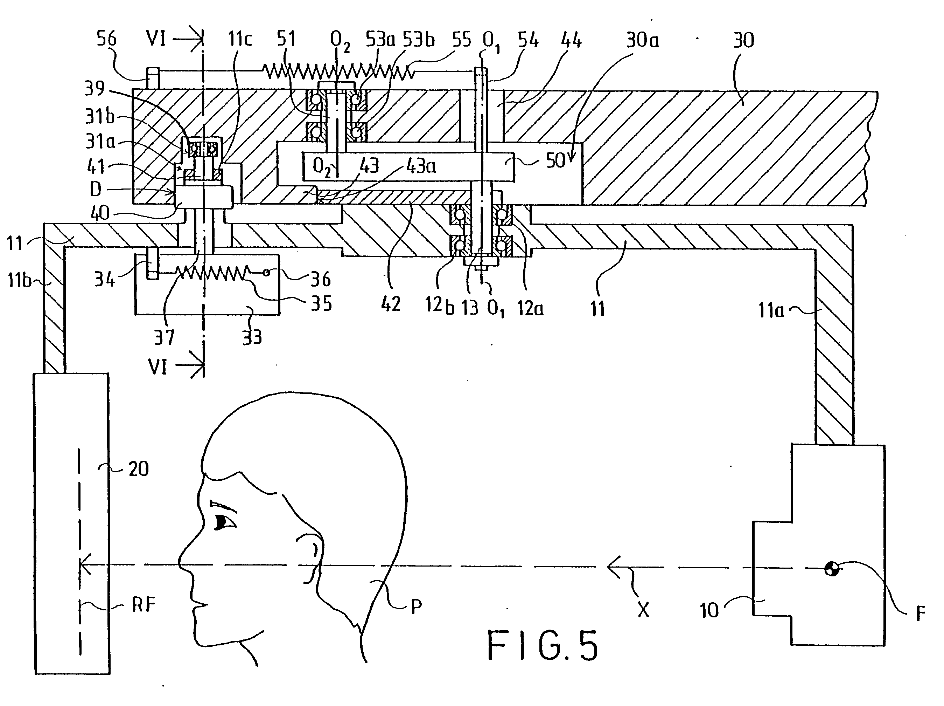

the distance $b_0$-$b_5$ is constant, the film 20 can be brought closer to the patient P (figure 5) in order to reduce the magnification ratio without a risk of the film cartridge hitting the patient. The described geometry also means an essentially lower level of radiation compared with the Prior Art, as the lateral centers of rotation of the frame 11 are located outside the patient.

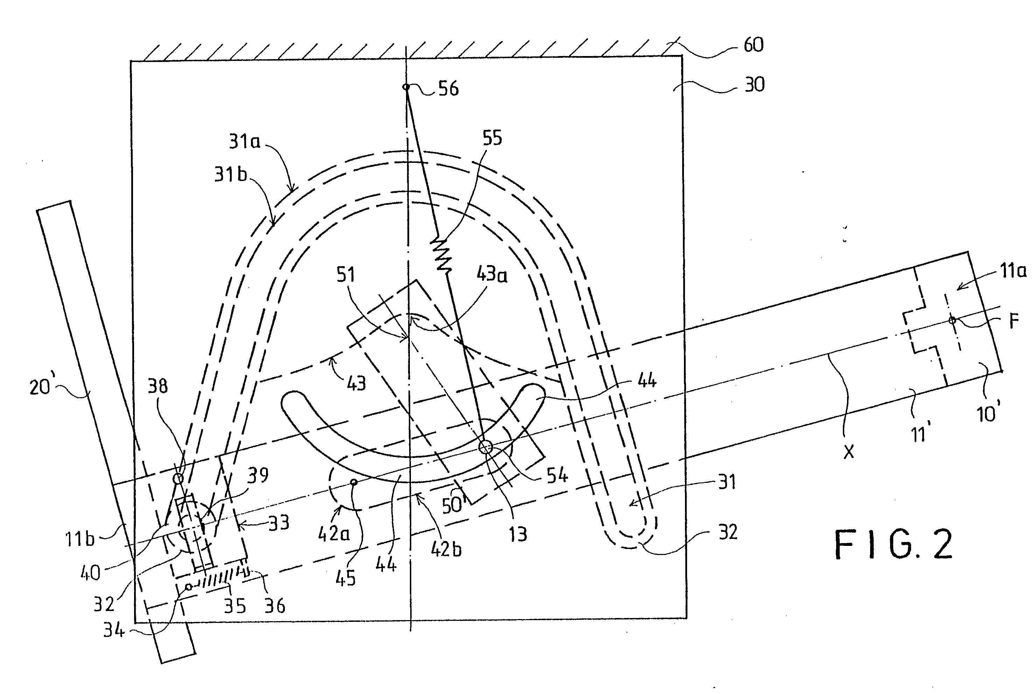

We shall now describe, with reference to figures 2 to 6, a mechanism of moving the X-ray tube 10 and the film 20 to accomplish the above-described representation projection and geometry. At first we shall describe the design of an apparatus in accordance with the invention with reference to figures 2 to 6 ; this is followed by a closer description of the design of the turning mechanism of the rotary frame 11 with reference to figures 2, 3 and 4, in which figures the turning mechanism is shown in different positions.

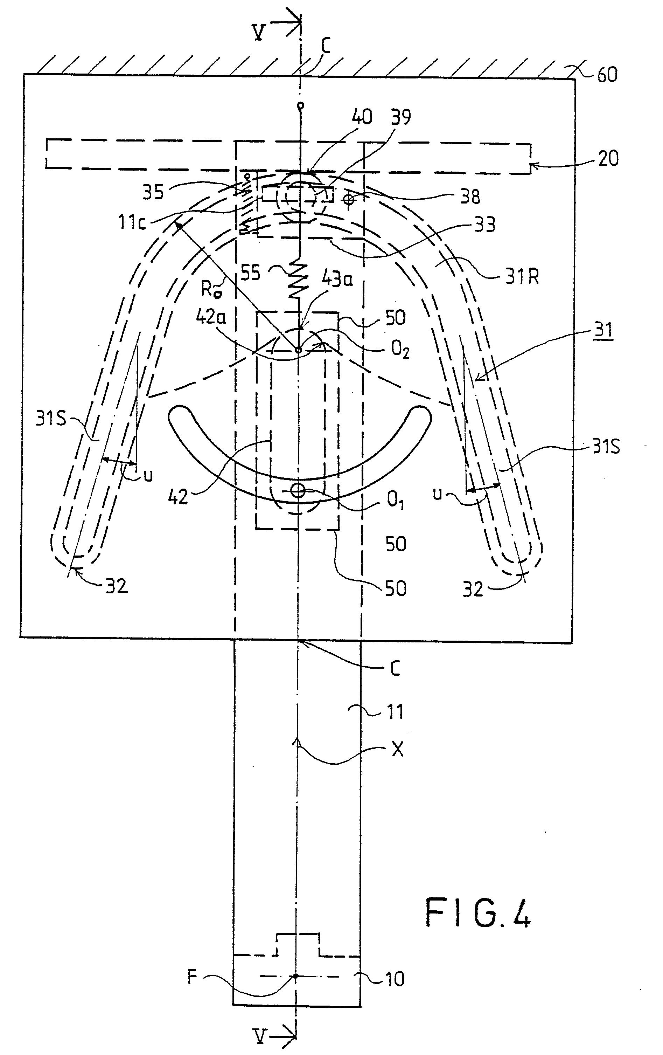

The turning mechanism comprises a frame section 30, which is fastened with a method known as such to a base 60 standing on the floor. The base may have a provision for moving the frame section 30 to a suitable working height. The rotary frame (arm) 11, rotated in the horizontal plane, is suspended to the base 30 by means of a mechanism in accordance with the invention ; as seen for example from figure 4, the rotary frame 11 is essentially an elongated arm, at whose one end, supported by a vertical part 11b, there is a film cartridge 20 containing X-ray film RF and a drive mechanism and other equipment known as such, not needing any closer description in this context. The rotary frame 11 is suspended by means of bearings 12a and 12b to be rotated around a vertical shaft $O_1$-$O_1$. The vertical shaft $O_1$-$O_1$ is not fixed but is, during the exposure, horizontally swung with regard to the fixed frame 30. A rear journal pin 13 of the rotary frame 11 is fixedly fastened to the X-ray-tube-end 10 of the crank 50. At the other end of the crank 50 turning in the space 30a of the frame 30 there is a front journal pin 51 installed with bearings 53a, 53b in association with the fixed frame 30. Both journal pins 13 and 51 have two opposite bevelled ball bearings 12a, 12b ; 53a, 53b, preloaded to be completely play-free and carrying both the axial loadings due to the weight of the rotary frame 11 and equipment attached to it and the radial forces during the rotation of the rotary frame 11. Instead of the bevelled ball bearings 12, 53 one may also use equivalent conical ball bearings or other similar combined axial and radial bearings. From the point of view of the rotary mechanism it is very important that the described bearing system is well designed and correctly built and installed.

In addition to the described crank 50 and its journal pins 13 and 51, a rotary mechanism also essentially comprises a counter-profile 42 which is fixedly fastened to the top side of the rotary frame 11 so that one end of the counter-profile is around the vertical shaft 13, and the round surface 43a of its other end is — when the apparatus is in the middle position shown in figure 4 — at the journal pin 51. A guide profile 43, which has vertical guiding surface shaped as illustrated by dotted lines in figures 2, 3 and 4, interacts with the round end surface 42a and the flat sides 42b of the counter-profile 42. Said guide profile 43 is symmetrical with respect to the center plane C-C, and in the middle of it there is a round recess 43a, against which the round end of the counter-profile 42 leans contributing to the stability of the mechanism when operating in its middle position shown in figure 4 and on both sides of this position. The interaction of the counter-profile 42 and the guide profile 43 in various operating positions will be described more closely below.

In a mechanism in accordance with the invention an essential feature is the equipment with which the rotating force is transmitted to the rotary frame 11. This equipment comprises a guide groove 31 located in the fixed frame 30 and opening to its underside, and having a shape essentially similar to the main shape of the jawbone and the dental arch taking into account the constant distance $b_0$-$b_5$ described in association with figure 1. The guide groove 31 is symmetrical with regard to the vertical center plane C-C, and it has side sections 31S, positioned at a narrow angle u with respect to the plane C-C and ending at end sections 32, and a curved section 31R connecting the side sections with each other. The curved section 31R has a section with a constant radius R whose center point is located on the vertical axis $O_2$, and two sections, on which the radius of curvature R changes and smoothly becomes infinitely large to turn into the straight sections 31S. In figure 1, one half of the constant-radius section is represented by sector c, and one of the sections with the growing radius of curvature is represented by sector d, on which the center of rotation moves from the axis $O_2$ to the axis $O_3$. Generally the angle u is in the range u = 10°-20°. In the guide groove there is a narrower bottom section 31b and a broader mouth section 31a, which sections have constant widths over the whole length of the groove 31.

The groove 31 works as a guide and drive block together with the drive unit 33 fastened to the rotary frame 11. As the drive motor, in the unit 33 there is an electrical motor and a transmission which turns the drive wheel 40 installed on the drive shaft 37 of the unit 33. The unit is suspended pivotably by means of a journal pin 38 located in association with the frame section 11. The unit 33 is turned around the journal pin 38 to the driving position of the drive wheel 40 by a spring 35 installed between the pins 34 and 36, which spring 35 gives a constant load to the drive wheel 40 particularly against the outer side of the drive (drawing) section 31a of the groove 31. In figure 5, the point where the drawing occurs is indicated with D.

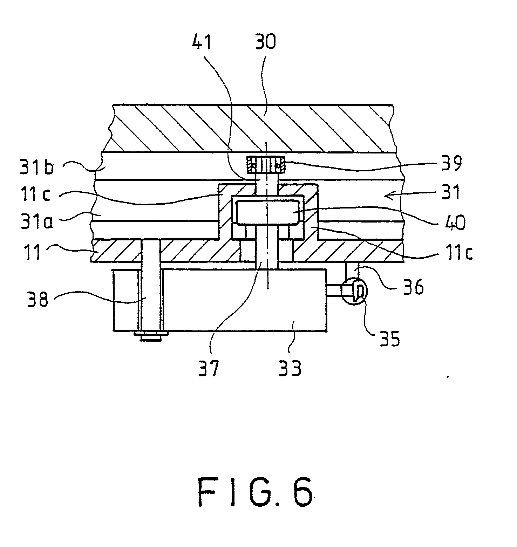

As shown in figures 5 and 6, the rotary frame 11 has, at the drive wheel 40, a protruding part 11c, which reaches on top of the drive wheel 40. A journal pin 41 is attached to the side of the protruding part 11c, at whose end there is a guide wheel 39, which is loaded so as to make it lean against the inner side of the guide section 31b of the groove 31 ; this inner side is opposite to the side against which the drive wheel 40 is loaded to lean on the section 31a. The drive wheel 40 and the guide wheel 39 are arranged so as to make their centers of rotation unite. Then the loading spring 35 will not cause any extra torque which might disturb the rotary movement.

Figures 2, 3 and 4 show a favourable location of the spring element 35 of the driving unit 33 : the spring element 35 is at the opposite end of the unit 33 with regard to the journal pin 33 preferably so that the spring 35 and the shaft 38 are located essentially symmetrically on both sides of the longitudinal center plane of the rotary frame 11.

An important feature of the described drive mechanism is that the drive unit 33 together with the loading spring 35 ensures that the drive wheel 40 in all positions of the rotary frame 11 follows the outer edge of the drive (drawing) section 31a of the groove 31 and the guide wheel 39 similarly follows play-freely the inner edge of the guiding section 31b, which provides a play-free, smooth, reliable and jerkless drive in all positions of the rotary frame 11.

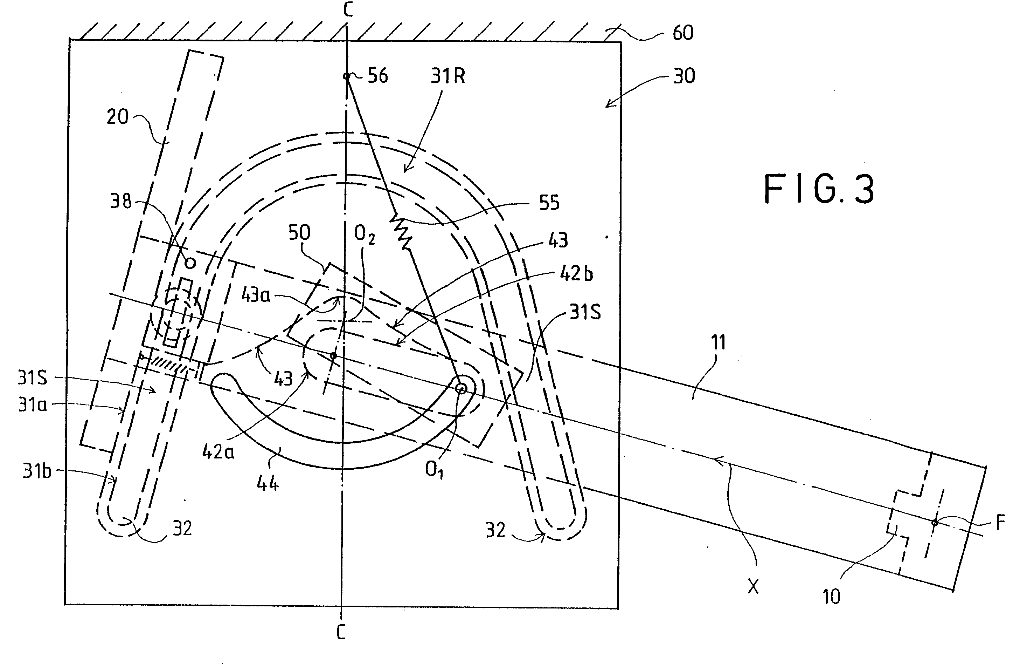

In the following we will, with reference to figures 2, 3 and 4, describe the operation of the turning crank mechanism and those details of its design which have not been mentioned above. This description is started from the middle position shown in figure 4, although the device usually starts moving from the end position 11' of the rotary frame 11, and the rotary frame 11 turns via the intermediate position shown in figure 3 to the middle position shown in figure 4 and then proceeds, guided by the right-hand section of the groove 31 to the opposite end position corresponding to figure 2. At both sides of the middle position shown in figure 4, in other words on the sector C between the radii $a_0$ and $a_2$ shown in figure 1, the rotary frame 11 is rotated around the rotary axis $O_2$ by the interaction of the curved, constant-radius section 31R of the groove 31 the drive wheel 40 and the guide wheel 39. In this position the crank 50 is parallel with the arm 11, and the rotary axes $O_1$ and $O_2$ of the journal pins 13 and 51 are located in the vertical middle plane C-C of the rotary frame 11. In order to make the middle position of the crank mechanism 11, 13, 50, 51 and 30 controlled and not vague, the mechanism comprises the counter-profile 42, whose guiding end 42a leans play-freely against the round recess 43a of the guide profile 43 of the fixed frame 30. Then the parts 42a and 43a will, on their part, define the stable center of rotation of the mechanism. When the mechanism begins to rotate from the middle position of figure 4 for instance towards the intermediate position showm in figure 3, the center of rotation $O_2$ determined by the parts 42a and 43a will stay on the constant-radius section 31R of the guide groove 31. The shape of the groove 31 resembles the shape of the jawbone.

When the guide wheel 39 and the drive wheel 40 come from the constant-radius $R_0$ section of the groove 31 to its growing-radius ($R_0$) section, the center of rotation of the rotary frame 11 begins to move from the center $O_2$ and the center plane C-C towards the side, which occurs so that the crank 50 begins to turn unparallel with respect to the rotary frame 11, and the straight vertical side 42b of the counter-profile 42 leans against the curved side of the guide profile 43 tangentially touching it and possibly sliding along it so that this tangent rolls and possibly also slides from the recess 43a of the guide profile 43 to the side section of the guide profile 43, whereat the center of rotation of the rotary frame 11 simultaneously continuously and smoothly moves from the axis $O_2$ to the axis $O_3$. On this area (sector d in figure 1) the rotary frame 11 and the crank 50 horizontally turn to the same direction. When moving from the position of figure 3 to the position of figure 2 (sector e in figure 1) the crank 50 turns to the direction that is opposite to the turning direction of the rotary frame 11. In the area of the straight guide grooves 31S the counter-profile does not work. In the front area of the dental arch, i. e. when the rotary frame 11 turns around the axis $O_2$, the exact location and smooth moving of the center of rotation from one control phase to another is ensured, besides by means of the counter-profile 42 and the guide profile 32, also by means of a guide spring 55. The guide spring 55 is positioned between a pin 54 protruding through the groove 44 of the fixed frame 30 and a pin 56 of the frame 30. The spring 55 ensures that the guide profile 43 and the counter-profile 52 at all times stay in stable contact with each other when operating in the area 31R of the groove 31.

It should be emphasized that what has been described above is only one feasible embodiment of the invention ; the details of this embodiment may vary in many ways within the scope of the invention. In lieu of the guide groove 31 it is possible to use for instance a guide rail, whose shape corresponds to the shape of the guide groove 31, and which guide rail may be arranged to work for instance so that the drive wheel 40 rolls against its one side, and the guide wheel 39 rolls against its other side. Stable drive and stable control may be also be. ensured with this method. The drive unit 33 may also be arranged in some other way than what is shown in the figures, and its spring loading may be created in may different ways. The details of the crank mechanism may also vary and differ from what was described above.

A mechanism in accordance with the invention provides a particularly feasible shape of the running path of the exposed area of the film RF being exposed, marked in figure A with a dotted line, and other advantages discussed above, which are synergically connected with both the operation of the device and its mechanical design.

We have described such an embodiment of the invention in which the drive groove 31 is located in the fixed section 30 and the drive unit 33 in the rotary frame (arm) 11. In some special occasions, even though this kind of design does not seem feasible nowh, it may be possible to carry out the guide and drive device so that the drive unit with its guide wheels is located in association with the fixed frame 30 and the guide groove or guide rail is arranged in association with the rotary frame 11, in which case the drive unit 33 and the drive groove or similar have changed their positions.

**Claims**

1. A panoramic tomography X-ray apparatus particularly for dental photography, which apparatus comprises a fixed frame (30), to which a rotary frame (11), having an X-ray tube (10) at one end and a film cartridge device (20) for X-ray film (RF) at the other end, is suspended with bearings (12a, 12b, 53a, 53b), and between which X-ray tube (10) and film cartridge (20) the patient's (P) object to be photographed can be positioned, and which apparatus has a drive mechanism by means of which said suspended rotary frame (11) is rotated in an horizontal plane, for taking a panoramic X-ray photograph, characterized in

that said drive device comprises a drive and guide groove (31) or a corresponding rail system, which is symmetrical with respect to a vertical center plane (C-C) of the apparatus, and which has essentially straight side sections (31S) and a curved section (31R) interconnecting the side sections, said drive and guide groove (31) having a shape essentially similar to the main shape of the jawbone and the dental arch,

that a drive unit (33) is installed to interact with said drive and guide groove (31), which drive unit comprises a drive wheel (40) which, rotated by the drive unit (33) moves along said drive and guide groove (31) and turns said rotary frame (11) around a vertical axis ($O_1$) and the patient, which vertical axis ($O_1$) is movable in the horizontal plane by means of a drive and guide mechanism (33-41),

that said rotary frame (11), to be rotatable in the horizontal plane, is suspended to said fixed frame (30) by means of a crank of the crank mechanism, joined to the rotary frame (11) by means of a bearing (12a, 12b) provided along axis ($O_1$), and at the opposite end, joined to the fixed frame (30) by means of another bearing (53a, 53b) provided along axis ($O_2$) so that, when the rotary frame (11) is in a middle position wherein said drive wheel (40) is placed in said vertical center plane (C-C), the two opposite joining axes ($O_1$, $O_2$) (journal pins) (13, 51) of the crank (50) are positioned in the vertical center plane (C-C) of the apparatus, and

that said drive and guide mechanism (33-41) and said crank mechanism are arranged to interact in such a way that, in the front area of the dental arch or similar, i. e. when the corresponding section (31R) of the guide and drive groove (31) is in operation, the rotary frame (11) turns only around the front journal pin (51), i. e. the journal pin located on the side of the X-ray film cartridge (20), and in the side areas of the dental arch or similar, when guided by the straight sections (31S) of the drive and guide groove (31), the rotary frame (11) simultaneously turns around both journal pins (13, 51) of the crank mechanism (13, 50, 51).

2. An apparatus in accordance with claim 1, characterized in that the guide groove (31) or similar is so shaped as to make the exposed portion of the X-ray film (RF) in the film cartridge (20) pass the object being photographed at a constant distance ($b_0$-$b_5$) so that the magnification ratio will essentially be constant over the whole image field.

3. An apparatus in accordance with claims 1 or 2, characterized in that a guiding block, preferably a guide wheel (39), is installed to interact with said drive wheel (40), which guide wheel (39) is installed in association with the rotary frame (11), preferably coaxially with the rotary axis of the drive wheel (40), and that said guiding block (39) is arranged to lean against that side of the drive and guide groove (31) which is opposite to the driving point (D) of the drive wheel (40).

4. An apparatus in accordance with one of claims 1 to 3, characterized in that both opposite journal pins (13, 51) of said crank (50) have two opposite bevelled ball bearings (12a, 12b ; 53a, 53b) or equivalent conical ball bearings, preferably preloaded to be completely play-free in such a way as to simultaneously carry both the axial loadings due to the weight of the equipment and the radial forces during the rotation.

5. An apparatus in accordance with one of claims 1 to 4, characterized in that said guide and drive groove (31) is arranged to open to the underside of said fixed frame (30), and that for interaction with said groove (31) such a drive and guide device is arranged into the rotary frame (11) that comprises a drive unit (33) turning around a vertical axis (38), in which drive unit (33) there is a spring element (35) or similar for loading the drive wheel (40) against the outer edge of said groove (31), and that a protruding part (11c) is fastened to the rotary frame (11), to which protruding part a guide wheel (39), coaxial with said drive wheel (40), is fastened, said guide wheel (39) being installed to lean against the vertical side of the drive and guide groove opposite to the touching point (D) of the drive wheel (40).

6. An apparatus in accordance with one of claims 1 to 5, characterized in that in said drive and guide groove (31) there is a bottom section (31b) functioning as the guide groove and a broader outer section (mouth section) (31a), which functions as the drive groove and is slightly broader than the diameter of the drive wheel (40).

7. An apparatus in accordance with one of claims 1 to 6, characterized in that the journal pin (38) and the spring element (35) of said drive unit (33) are arranged on the opposite sides of the passing point of the X-ray (X) so that said spring element (35) loads said unit (33) towards the X-ray film cartridge (20) and towards the outer side of the guide groove (31a).

8. An apparatus in accordance with one of claims 1 to 7, characterized in that said crank mechanism (13, 50, 51) comprises a counter-profile (42) fastened to the top side of the rotary frame (11) that turns, when the crank is in its middle position, under the crank, and a guide profile (43) fastened to the opposite bottom side of the fixed frame (30), which profiles (42, 43) have symmetrical guide surface, (42a, 42b) being in the center position of the rotary frame (11) and in said center plane (C-C), symmetrically with regard to said center plane (C-C), which guide surfaces (42a, 42b) are, in the above position, concentric with the rotary axis ($O_2$) of the X-ray-film-side journal pin (51), and which guide surfaces precisely determine the position of the instantaneous rotary centre in the curved middle area of the drive and guide groove (31), and that the straight side (42b) of said counter-profile (42) and the curved side of said guide profile (43), when touching each other, move the rotary centre thus increasing the turning radius of the rotary frame (11).

9. An apparatus in accordance with one of claims 1 to 8, characterized in that said crank (50) is connected to a spring element (55), which ensures the mutual supporting contact between the guiding surface (42b) of the counter-profile (42) and the curved guiding surface of the guide profile (43) and the exact location and smooth moving of the rotary centre of the rotary frame (11) from the front journal pin (51) of the crank towards the side.

10. An apparatus in accordance with claim 9, characterized in that in said crank (50), at its rear journal pin (13), there is a pin (54) which reaches through the groove (44) of the fixed frame (30) outside the top side of said fixed frame (30), and to which pin (54) such a spring (55) is fastened whose other end is fastened to said fixed frame (30).

11. An apparatus in accordance with one of claims 1 to 10, characterized in that said drive wheel (40) and guide wheel (39) are coaxially positioned on the same vertical shaft so that the rotary frame (11) may move without any axial loading disturbing the smooth, play-free and jerkless drive of the drive wheel (40).

## Patentansprüche

1. Panorama-Tomografie-Röntgenstrahlenvorrichtung, insbesondere für die Dental-Fotografie, mit einem feststehenden Rahmen (30), an dem ein rotierender Rahmen (11), der eine Röntgenröhre (10) an einem Ende und eine Filmpatroneneinrichtung (20) für einen Röntgenfilm (RF) am anderen Ende aufweist, mittels Lager (12a, 12b, 53a, 53b) aufgehängt ist, wobei zwischen der Röntgenröhre (10) und der Filmpatrone (20) das zu fotografierende Patientenobjekt (P) positioniert werden kann, und mit einem Antriebsmechanismus, durch den der genannte aufgehängte rotierende Rahmen (11) in einer horizontalen Ebene für die Aufnahme einer Panorama-Röntgenaufnahme gedreht wird, dadurch gekennzeichnet,

daß die Antriebseinrichtung eine Antriebs- und Führungsnut (31) oder ein entsprechendes Schienensystem aufweist, welches symmetrisch in bezug auf eine vertikale Mittelebene (C-C) der Vorrichtung ist und welches im wesentlichen gerade Seitenabschnitte (31S) und einen gekrümmten Abschnitt (31R) aufweist, der die Seitenabschnitte miteinander verbindet, daß die Antriebs- und Führungsnut (31) eine Form aufweist, die im wesentlichen der Hauptform des Kieferknochens und des Zahnbogens ähnlich ist,

daß eine Antriebseinheit (33) derart installiert ist, daß sie mit der Antriebs- und Führungsnut (31) zusammenwirkt, daß die Antriebseinheit (33) ein Antriebsrad (40) aufweist, welches durch die Antriebseinheit (33) in Drehung versetzt sich längs der genannten Antriebs- und Führungsnut (31) bewegt und den rotierenden Rahmen (11) um eine vertikale Achse ($O_1$) und den Patienten dreht, wobei die vertikale Achse ($O_1$) in der horizontalen Ebene mittels eines Antriebs- und Führungsmechanismus (33-41) bewegbar ist,

daß der rotierende Rahmen (11), um in der horizontalen Ebene drehbar zu sein, an dem feststehenden Rahmen (30) mittels einer Kurbel des Kurbelmechanismus aufgehängt, mit dem rotierenden Rahmen (11) mittels eines Lagers (12a, 12b) verbunden, welches längs der Achse ($O_1$) vorgesehen ist, und am gegenüberliegenden Ende mit dem feststehenden Rahmen (30) mittels eines weiteren Lagers (53a, 53b) verbunden ist, welches längs einer Achse ($O_2$) vorgesehen ist, derart, daß dann, wenn der rotierende Rahmen (11) sich in einer Mittelstellung befindet, in der das Antriebsrad (40) in der genannten vertikalen Mittelebene (C-C) angeordnet ist, die beiden gegenüberliegenden Verbindungsachsen ($O_1$, $O_2$) (Lagerzapfen) (13, 51) der Kurbel (50) in der vertikalen Mittelebene (C-C) der Vorrichtung Positioniert sind,

und daß der genannte Antriebs- und Führungsmechanismus (33-41) und der Kurbelmechanismus so angeordnet sind, daß sie in einer solchen Weise miteinander zusammenwirken, daß im vorderen Bereich des Zahnbogens oder dergleichen, das heißt dann, wenn der entsprechende Abschnitt (31R) der Führungsund Antriebsnut (31) in Betrieb ist, der rotierende Rahmen (11) sich lediglich um den vorderen Lagerzapfen (51) dreht, das heißt um den Lagerzapfen, der auf der Seite der Röntgenfilmpatrone (20) angeordnet ist, und daß in den Seitenbereichen des Zahnbogens oder dergleichen, wenn eine Führung durch die geraden Abschnitte (31S) der Antriebs- und Führungsnut (31) erfolgt, der rotierende Rahmen (11) sich gleichzeitig um beide Lagerzapfen (13, 51) des Kurbelmechanismus (13, 50, 51) dreht.

2. Vorrichtung nach Anspruch 1, dadurch gekennzeichnet, daß die Führungsnut (31) oder dergleichen so geformt ist, daß der freigelegte Teil des Röntgenfilms (RF) in der Filmpatrone (20) sich an dem fotografierten Gegenstand in einer konstanten Entfernung ($b_0$-$b_5$) vorbeibewegt, so

daß das Vergrößerungsverhältnis über das gesamte Bildfeld im wesentlichen konstant ist.

3. Vorrichtung nach Anspruch 1 oder 2, dadurch gekennzeichnet, daß ein Führungsblock, vorzugsweise ein Führungsrad (39), derart installiert ist, daß es mit dem genannten Antriebsrad (40) zusammenwirkt, daß das Führungsrad (39) in Verbindung mit dem rotierenden Rahmen (11) vorzugsweise koaxial zur Rotationsachse des Antriebsrades (40) installiert ist, und daß der genannte Führungsblock (39) so angeordnet ist, daß er gegen diejenige Seite der Antriebs- und Führungsnut (31) abgestützt ist, die dem Antriebspunkt (D) des Antriebsrades (40) gegenüberliegt.

4. Vorrichtung nach einem der Ansprüche 1 bis 3, dadurch gekennzeichnet, daß die beiden gegenüberliegenden Lagerzapfen (13, 51) der genannten Kurbel (50) zwei gegenüberliegende abgeschrägte Kugellager (12a, 12b, 53a, 53b) oder äquivalente konische Kugellager aufweist, die, um vollständig spielfrei zu sein, vorzugsweise in einer solchen Weise vorgespannt sind, daß gleichzeitig sowohl die axialen Belastungen aufgrund des Gewichts der Anordnung als auch die radialen Kräfte während der Drehung aufgenommen sind.

5. Vorrichtung nach einem der Ansprüche 1 bis 4, dadurch gekennzeichnet, daß die genannte Führungs- und Antriebsnut (31) so angeordnet ist, daß sie zur Unterseite des feststehenden Rahmens (30) hin offen ist, und daß zum Zusammenwirken mit der genannten Nut (31) eine solche Antriebs- und Führungseinrichtung in dem rotierenden Rahmen (11) angeordnet ist, welche eine Antriebseinheit (33) umfaßt, die sich um eine vertikale Achse (38) dreht und in der ein Federelement (35) oder dergleichen zur Belastung des Antriebsrades (40) gegen die Außenkante der genannten Nut (31) vorgesehen ist, und daß an dem rotierenden Rahmen (11) ein vorstehender Teil (11c) befestigt ist, an dem ein koaxial zu dem genannten Antriebsrad (40) verlaufendes Führungsrad (39) befestigt ist, welches so installiert ist, daß es sich gegen die vertikale Seite der Antriebs- und Führungsnut gegenüber dem Berührungspunkt (D) des Antriebsrades (40) abstützt.

6. Vorrichtung nach einem der Ansprüche 1 bis 5, dadurch gekennzeichnet, daß in der genannten Antriebs- und Führungsnut (31) ein als Führungsnut wirkender Bodenabschnitt (31b) und ein breiterer Außenabschnitt (Mundabschnitt) (31a) vorgesehen sind, der als Führungsnut wirkt und etwas breiter ist als der Durchmesser des Antriebsrades (40).

7. Vorrichtung nach einem der Ansprüche 1 bis 6, dadurch gekennzeichnet, daß der Lagerzapfen (38) und das Federelement (35) der genannten Antriebseinheit (33) auf den gegenüberliegenden Seiten des Durchgangspunktes der Röntgenstrahlen (X) derart angeordnet sind, daß das genannte Federelement (35) die genannte Einheit (33) zu der Röntgenfilmpatrone (20) und zur Außenseite der Führungsnut (31a) hin belastet.

8. Vorrichtung nach einem der Ansprüche 1 bis 7, dadurch gekennzeichnet, daß der genannte Kurbelmechanismus (13, 50, 51) ein an der Oberseite des rotierenden Rahmens (11) befestigtes Gegenprofil (42) aufweist, welches sich dann, wenn die Kurbel sich in ihrer Mittelstellung befindet, unter die Kurbel dreht, und ein Führungsprofil (43) umfaßt, welches an der gegenüberliegenden Bodenseite des feststehenden Rahmens (30) befestigt ist, wobei die Profile (42, 43) symmetrische Führungsflächen (42a, 42b) aufweisen, die in der Mittelstellung des rotierenden Rahmens (11) und in der genannten Mittelebene (C-C) symmetrisch in bezug auf die genannte Mittelebene (C-C) sind, daß die Führungsflächen (42a, 42b) in der oberen Stellung konzentrisch zur Rotationsachse $(O_2)$ des Lagerzapfens (51) auf der Seite des Röntgenfilms sind, daß die Führungsflächen die Position der momentanen Rotationsmitte im gekrümmten mittleren Bereich der Antriebs- und Führungsnut (31) genau festlegen und daß die gerade Seite (42b) des Gegenprofils (42) und die gekrümmte Seite des Führungsprofils (43) bei Berührung aneinander die Rotationsmitte bewegen, womit der Drehradius des rotierenden Rahmens (11) vergrößert wird.

9. Vorrichtung nach einem der Ansprüche 1 bis 8, dadurch gekennzeichnet, daß die genannte Kurbel (50) mit einem Federelement (55) verbunden ist, das den gegenseitigen Tragkontakt zwischen der Führungsfläche (42b) des Gegenprofils (42) und der gekrümmten Führungsfläche des Führungsprofils (43) sowie die exakte Lage und die gleichmäßige Bewegung der Rotationsmitte des rotierenden Rahmens (11) von dem vorderen Lagerzapfen (51) der Kurbel zur Seite hin sicherstellt.

10. Vorrichtung nach Anspruch 9, dadurch gekennzeichnet, daß in der genannten Kurbel (50) an deren hinteren Lagerzapfen (13) ein Zapfen (54) vorhanden ist, der durch die Nut (44) des feststehenden Rahmens (30) außerhalb der Deckseite des betreffenden feststehenden Rahmens (30) ragt und an dem eine solche Feder (55) befestigt ist, deren anderes Ende an dem feststehenden Rahmen (30) befestigt ist.

11. Vorrichtung nach einem der Ansprüche 1 bis 10, dadurch gekennzeichnet, daß das genannte Antriebsrad (40) und das Führungsrad (39) koaxial auf derselben vertikalen Welle derart positioniert sind, daß der rotierende Rahmen (11) sich ohne irgendeine den gleichmäßigen, spielfreien und ruckfreien Antrieb des Antriebsrades (40) störende axiale Belastung bewegen kann.

## Revendications

1. Un appareil à rayons X pour la tomographie panoramique, et en particulier pour la radiographie dentaire, cet appareil comprenant un châssis fixe (30) sur lequel un châssis tournant (11) est suspendu au moyen de paliers (12a, 12b, 53a, 53b), le châssis tournant portant un tube à rayons X (10) à une extrémité et une cassette de film (20) pour un film sensible aux rayons X (RF) à l'autre extrémité, la partie du patient (P) à radiographier pouvant être positionnée entre le tube à rayons X (10) et la cassette de film (20), et cet appareil comportant un mécanisme d'entraînement au moyen duquel le châssis tournant suspendu (11) est mis en rotation dans un plan horizontal pour prendre une radiographie panoramique, caractérisé en ce que :

le dispositif d'entraînement comprend une rainure d'entraînement et de guidage (31) ou un système de rails correspondants, qui est symétrique par rapport à un plan central vertical (C-C) de l'appareil, et qui comporte des sections latérales pratiquement rectilignes (31S) et une section courbe (31R) qui relie les sections latérales, la rainure d'entraînement et de guidage (31) ayant une forme pratiquement similaire à la forme principale du maxillaire et de l'arc dentaire,

en ce qu'un organe d'entraînement (33) est installé de façon à interagir avec la rainure d'entraînement et de guidage (31), cet organe d'entraînement comprenant une roue d'entraînement (40) qui est mise en rotation par l'organe d'entraînement (33), se déplace le long de la rainure d'entraînement et de guidage (31) et fait tourner le châssis tournant (11) autour d'un axe vertical ($O_1$) et du patient, cet axe vertical ($O_1$) étant mobile dans le plan horizontal au moyen d'un mécanisme d'entraînement et de guidage (33-41),

en ce que, dans le but de pouvoir tourner dans le plan horizontal, le châssis tournant (11) est suspendu au châssis fixe (30) au moyen d'une manivelle d'un mécanisme à manivelle, reliée au châssis tournant (11) au moyen d'un palier (12a, 12b) centré sur l'axe ($O_1$), et relié au châssis fixe (30), à l'extrémité opposée, au moyen d'un autre palier (53a, 53b) centré sur l'axe ($O_2$), de façon que lorsque le châssis tournant (11) se trouve dans une position médiane, dans laquelle la roue d'entraînement (40) se trouve dans le plan central vertical (C-C), les deux axes de liaison opposés (manetons) (13, 51) de la manivelle (50) se trouvent dans le plan central vertical (C-C) de l'appareil, et

en ce que le mécanisme d'entraînement et de guidage (33-41) et le mécanisme à manivelle sont conçus pour interagir d'une manière telle que, dans la partie avant de l'arc dentaire ou dans une position similaire, c'est-à-dire lorsque la section correspondante (31R) de la rainure d'entraînement et de guidage (31) est en fonction, le châssis tournant (11) tourne seulement autour du maneton avant (51), c'est-à-dire autour du maneton qui se trouve du côté de la cassette de film pour rayons X (20), tandis que dans les zones latérales de l'arc dentaire ou dans des positions similaires, lorsque le guidage est effectué par les sections rectilignes (31S) de la rainure d'entraînement et de guidage (31), le châssis tournant (11) tourne simultanément autour des deux manetons (13, 51) du mécanisme à manivelle (13, 50, 51).

2. Un appareil selon la revendication 1, caractérisé en ce que la rainure de guidage (31) ou une structure similaire a une forme telle que la partie exposée du film pour rayons X (RF) dans la cassette de film (20) passe devant l'objet radiographié à une distance constante ($b_0$-$b_5$), ce qui fait que le rapport d'agrandissement est pratiquement constant sur tout le champ de l'image.

3. Un appareil selon les revendications 1 ou 2, caractérisé en ce qu'un bloc de guidage, consistant de préférence en une roue de guidage (39), est installé de façon à interagir avec la roue d'entraînement (40), et cette roue de guidage (39) est installée en association avec le châssis tournant (11), de préférence en position coaxiale avec l'axe de rotation de la roue d'entraînement (40), et en ce que le bloc de guidage (39) est conçu de façon à porter contre le côté de la rainure d'entraînement et de guidage (31) qui se trouve à l'opposé du point d'entraînement (D) de la roue d'entraînement (40).

4. Un appareil selon l'une des revendications 1 à 3, caractérisé en ce que les deux manetons opposés (13, 51) de la manivelle (50) sont équipés de deux roulements à billes à contact oblique opposés (12a, 12b ; 53a, 53b), ou de roulements à billes coniques équivalents, de préférence préchargés pour être entièrement dépourvus de jeu, de manière à supporter simultanément les charges axiales qui sont dues au poids de l'équipement et les forces radiales pendant la rotation.

5. Un appareil selon l'une quelconque des revendications 1 à 4, caractérisé en ce que la rainure d'entraînement et de guidage (31) est conçue de façon à s'ouvrir à la face inférieure du châssis fixe (30), et en ce que, pour l'interaction avec la rainure (31) un dispositif d'entraînement et de guidage est installé dans le châssis tournant (11) et comprend un organe d'entraînement (33) qui tourne autour d'un axe vertical (38), cet organe d'entraînement (33) contenant un élément à ressort (35) ou similaire, destiné à faire en sorte que la roue d'entraînement (40) porte contre le bord extérieur de la rainure (31), et en ce qu'un élément en saillie (11c) est fixé sur le châssis tournant (11), et une roue de guidage (39), coaxiale par rapport à la roue d'entraînement (40), est fixée sur cet élément en saillie, et cette roue de guidage (39) est installée de façon à porter contre le côté vertical de la rainure d'entraînement et de guidage qui est opposé au point de contact (D) de la roue d'entraînement (40).

6. Un appareil selon l'une des revendications 1 à 5, caractérisé en ce que la rainure d'entraînement et de guidage (31) comporte une section de fond (31b) qui remplit la fonction de la rainure de guidage, et une section extérieure plus large (embouchure) (31a), qui remplit la fonction de la rainure d'entraînement et qui a une largeur légèrement supérieure au diamètre de la roue d'entraînement (40).

7. Un appareil selon l'une des revendications 1 à 6, caractérisé en ce que l'axe (38) et l'élément à ressort (35) de l'organe d'entraînement (33) sont disposés de part et d'autre du point de passage des rayons X (X), de façon que l'élément à ressort (35) sollicite l'organe précité (33) vers la cassette de film pour rayons X (20), et vers le côté extérieur de la rainure de guidage (31a).

8. Un appareil selon l'une des revendications 1 à 7, caractérisé en ce que le mécanisme à manivelle (13, 50, 51) comprend un contre-profil (42) qui est fixé au côté supérieur du châssis tournant (11), qui tourne sous la manivelle, lorsque la manivelle est dans sa position médiane, et un profil de guidage (43) qui est fixé au côté inférieur opposé du châssis fixe (30), ces profils (42, 43) ont des surfaces de guidage symétriques (42a, 42b) qui sont symétriques par rapport au plan central (C-C), dans la position centrale du châssis tournant (11) dans le plan central (C-C), dans la position ci-dessus, les surfaces de guidage (42a, 42b) sont concentriques par rapport à l'axe de rotation ($O_2$) du maneton du côté du film pour rayons X (51), et ces surfaces de guidage déterminent de façon précise la position du centre de rotation instantanée dans la zone médiane courbe de la rainure d'entraînement et de guidage (31), et en ce que le côté rectiligne (42b) du contre-profil (42) et le côté courbe du profil de guidage (43), lorsqu'ils se touchent, déplacent le centre de rotation, ce qui augmente le rayon de rotation du châssis tournant (11).

9. Un appareil selon l'une des revendications 1 à 8, caractérisé en ce que la manivelle (50) est reliée à un élément à ressort (55) qui assure le contact de support mutuel entre la surface de guidage (42b) du contre-profil (42) et la surface de guidage courbe du profil de guidage (43), et assure le positionnement précis et le déplacement progressif du centre de rotation du châssis tournant (11) à partir du maneton avant (51) de la manivelle, vers le côté.

10. Un appareil selon la revendication 9, caractérisé en ce qu'au niveau de son maneton arrière (13), la manivelle (50) comporte une tige (54) qui traverse la rainure (44) du châssis fixe (30), et sort par le côté supérieur du châssis fixe (30), et cette tige (54) est fixée à un ressort (55) dont l'autre extrémité est fixée au châssis fixe (30).

11. Un appareil selon l'une des revendications 1 à 10, caractérisé en ce que la roue d'entraînement (40) et la roue de guidage (39) sont montées de façon coaxiale sur le même axe vertical, de façon que le châssis tournant (11) puisse se déplacer sans qu'aucune charge axiale ne perturbe l'entraînement progressif, sans jeu et sans à-coups de la roue d'entraînement (40).

FIG.1

EP 0 204 676 B1

FIG. 2

FIG.3

FIG.4

FIG.5

EP 0 204 676 B1

FIG. 6